(19) Europäisches Patentamt
European Patent Office
Office européen des brevets

(11) Numéro de publication : **0 532 398 A1**

(12) # DEMANDE DE BREVET EUROPEEN

(21) Numéro de dépôt : **92402440.9**

(51) Int. Cl.$^5$ : **C07D 211/26,** A61K 31/445

(22) Date de dépôt : **07.09.92**

(30) Priorité : **12.09.91 FR 9111247**

(43) Date de publication de la demande : **17.03.93 Bulletin 93/11**

(84) Etats contractants désignés : **AT BE CH DE DK ES FR GB GR IE IT LI LU MC NL PT SE**

(71) Demandeur : **SYNTHELABO**
**22, Avenue Galilée**
**F-92350 Le Plessis Robinson (FR)**

(72) Inventeur : **George, Pascal**
**19, rue des Quatre Vents**
**F-78730 St Arnoult en Yvelines (FR)**
Inventeur : **Sevrin, Mireille**
**73, rue Raymond Losserand**
**F-75014 Paris (FR)**
Inventeur : **Froissant, Jacques**
**Cidex 981, Brevainville**
**F-41160 Morée (FR)**
Inventeur : **Maloizel, Christian**
**21 rue du Plateau**
**F-92190 Meudon (FR)**

(74) Mandataire : **Thouret-Lemaitre, Elisabeth et al**
**SYNTHELABO Service Brevets 22, avenue Galilée, B.P. Box 72**
**F-92352 LE PLESSIS ROBINSON CEDEX (FR)**

(54) **Dérivés de N-(4,7-diméthoxyindan-2-yl)-1-(phénylcarbonyl)-N-propyl-pipéridine-4-méthanamine, leur préparation et leur application en thérapeutique.**

(57) Composés de formule générale (I)

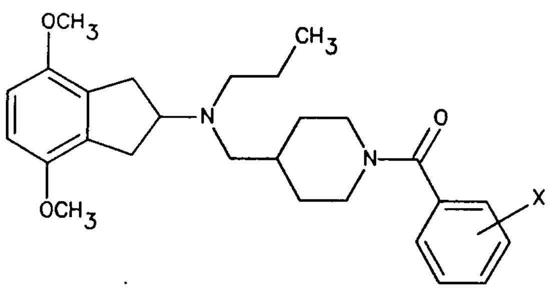

(I)

dans laquelle X représente un atome de chlore ou un groupe méthyle, méthoxy ou éthoxy.
Application en thérapeutique.

EP 0 532 398 A1

La présente invention a pour objet des dérivés de *N*-(4,7-diméthoxyindan-2-yl)-1-(phénylcarbonyl)-*N*-propyl-pipéridine-4-méthanamine, leur préparation et leur application en thérapeutique.

Les composés de l'invention répondent à la formule générale (I) du schéma donné ci-après, formule dans laquelle X représente un atome de chlore ou un groupe méthyle, méthoxy ou éthoxy.

Ils peuvent se présenter à l'état de bases libres ou de sels d'addition à des acides.

Un procédé de préparation des composés de l'invention est illustré par le schéma donné ci-après.

On fait réagir d'abord la 4,7-diméthoxyindan-2-amine de formule (II), éventuellement sous. forme de sel d'addition avec un acide, avec le chlorure de propionyle, dans un solvant aprotique inerte tel que le toluène, le tétrahydrofurane, ou un solvant halogéné, par exemple le dichlorométhane, et en présence d'une base organique telle qu'une amine tertiaire, par exemple la triéthylamine.

On obtient un amide qui, par réduction, par exemple avec l'hydrure de lithium et d'aluminium, dans un solvant éthéré tel que le tétrahydrofurane, fournit l'amine secondaire de formule (III).

On fait ensuite réagir celle-ci avec un tosylate de formule générale (IV) (dans laquelle Ts représente un groupe (4-méthylphényl)sulfonyle et X est tel que défini ci-dessus), soit en l'absence, soit en présence d'un solvant inerte tel que le diméthylformamide, le toluène ou le xylène, à une température de 20 à 150°C, et éventuellement en présence d'une base organique , telle qu'une amine tertiaire, ou minérale, telle qu'un carbonate ou hydrogénocarbonate alcalin.

La 4,7-diméthoxyindan-2-amine de formule (II) peut être préparée selon la méthode décrite dans *Can. J. Chem.*, **52**, 381-389 (1974).

## Schéma

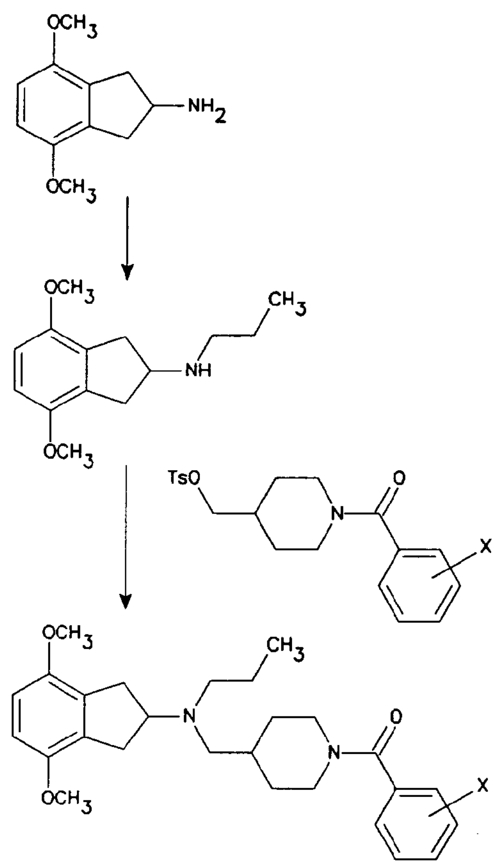

(II)

(III)

(IV)

(I)

Le tosylate de formule générale (IV) peut être préparé par exemple comme décrit dans la demande de brevet EP-0306375.

Les exemples suivants illustrent en détail la préparation de quelques composés selon l'invention.

Les microanalyses élémentaires et les spectres IR et RMN confirment les structures des produits obtenus.

Exemple 1

*N*-(4,7-diméthoxyindan-2-yl)-1-[(3-éthoxyphényl)carbonyl]-*N*-propyl-pipéridine-4-méthanamine, chlorhydrate.

1.1. *N*-(4,7-diméthoxyindan-2-yl)propanamide.

Dans un ballon tricol de 1 l on introduit 14 g (0,0724 mole) de 4,7-diméthoxyindan-2-amine, 400 ml de dichlorométhane et 11,1 ml (0,0796 mole) de triéthylamine puis, tout en agitant le mélange à température ambiante, on ajoute 6,6 ml (0,076 mole) de chlorure de propanoyle en solution dans 100 ml de dichlorométhane.

On agite le mélange pendant 3h et on le laisse reposer une nuit. On le lave trois fois à l'eau, on le sèche sur sulfate de sodium, on le filtre, on évapore le solvant sous pression réduite et on purifie le résidu par chromatographie sur colonne de gel de silice en éluant avec un mélange 98/2 de dichlorométhane/méthanol. On obtient 13,6 g de composé.

Point de fusion : 171-173°C.

1.2. 4,7-diméthoxy-*N*-propyl-indan-2-amine, chlorhydrate. Dans un flacon de Keller de 1 l on introduit, sous argon, 4,1 g (0,108 mole) d'hydrure de lithium et d'aluminium en suspension dans 200 ml de tétrahydrofurane, puis on ajoute, goutte à goutte, une solution de 13,6 g (0,054 mole) de *N*-(4,7-diméthoxyindan-2-yl)propanamide dans 300 ml de tétrahydrofurane, et on chauffe le mélange au reflux pendant 1h.

On le refroidit par un bain de glace, on ajoute 28,7 ml d'hydroxyde de sodium 1N, on sépare l'insoluble par filtration, et on évapore le filtrat sous pression réduite.

On obtient 13,1 g d'amine dont on prélève 1 g qu'on reprend avec 42 ml d'acide chlorhydrique 0,1N dans le propan-2-ol. On concentre la solution aux deux tiers et on la laisse cristalliser. On sépare les cristaux par filtration, on les dissout dans un peu de méthanol et on ajoute de l'éther diéthylique. Après filtration et séchage sous vide on obtient 0,8 g de chlorhydrate sous forme de cristaux blancs.

Point de fusion : 206-209°C.

1.3. *N*-(4,7-diméthoxyindan-2-yl)-1-[(3-éthoxyphényl)carbonyl]-*N*-propyl-pipéridine-4-méthanamine, chlorhydrate.

Dans un flacon de Keller de 25 ml on introduit, sous argon, 3 g (0,0127 mole) de 4,7-diméthoxy-*N*-propyl-indan-2-amine et 5,32 g (0,0127 mole) de 4-méthylbenzènesulfonate de 1-[(3-éthoxyphényl)carbonyl]pipéridin-4-ylméthyle, et on chauffe le mélange tout en l'agitant à 110°C pendant 3h.

On le laisse refroidir, puis on le reprend avec un mélange de dichlorométhane, d'eau et d'ammoniaque, on sépare la phase organique, on la lave trois fois à l'eau, on la sèche sur sulfate de magnésium, on la filtre et on évapore le solvant sous pression réduite. On purifie le résidu par chromatographie sur colonne de gel de silice en éluant avec un mélange 98/2 de dichlorométhane/méthanol. On obtient 1,6 g de composé sous forme de base.

On dissout celle-ci dans 33 ml de solution d'acide chlorhydrique 0,1N dans du propan-2-ol, on concentre la solution aux deux tiers, on ajoute de l'éther diéthylique et on sépare le précipité par filtration. On isole finalement 1,5 g de chlorhydrate.

Point de fusion : 160-164°C.

Exemple 2

*N*-(4,7-diméthoxyindan-2-yl)-1-[(3-chlorophényl)carbonyl]-*N*-propyl-pipéridine-4-méthanamine, chlorhydrate.

Dans un flacon de Keller de 25 ml on introduit, sous argon, 3 g (0,0127 mole) de 4,7-diméthoxy-*N*-propyl-indan-2-amine et 7,75 g (0,0190 mole) de 4-méthylbenzènesulfonate de 1-[(3-chlorophényl)carbonyl]pipéridin-4-ylméthyle, et on chauffe le mélange tout en l'agitant à 120°C pendant 10h.

On le laisse refroidir, puis on le reprend avec un peu de dichlorométhane, on le dilue avec de l'éther diéthylique et de l'eau, on ajoute de l'ammoniaque concentrée, on sépare la phase organique, on la lave à l'eau et on extrait le produit avec une solution d'acide chlorhydrique 1N. On lave la phase aqueuse acide avec de l'éther diéthylique, on ajoute de l'ammoniaque concentrée et on extrait le mélange trois fois avec de l'acétate d'éthyle. On lave la phase organique à l'eau, on la sèche sur sulfate de magnésium et on évapore le solvant sous pression réduite.

On obtient 3,8 g de produit huileux qu'on purifie par chromatographie sur colonne de gel de silice en éluant avec un mélange 98/2 de dichlorométhane/méthanol.

On obtient 1,3 g de base qu'on reprend dans un peu de propan-2-ol, on ajoute 27 ml de solution d'acide chlorhydrique 0,1N dans le propan-2-ol, puis de l'éther diéthylique, et on sépare le précipité par filtration. Après séchage on isole finalement 1,4 g de chlorhydrate.

Point de fusion : 192-198°C.

Le tableau qui suit illustre les structures chimiques et les propriétés physiques de quelques composés selon l'invention.

### Tableau

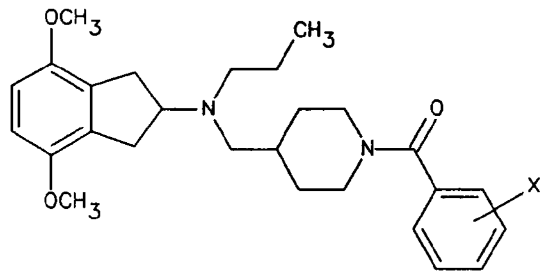

(I)

| N° | X | Sel | F (°C) |
|---|---|---|---|
| 1 | 3-Cl | chlorhydrate | 192-198 |
| 2 | $3-CH_3$ | chlorhydrate | 215-217 |
| 3 | $3-OCH_3$ | chlorhydrate | 194-198 |
| 4 | $3-OC_2H_5$ | chlorhydrate | 160-164 (déc.) |

Les composés de l'invention ont été soumis a une série d'essais pharmacologiques qui ont mis en évidence leur intérêt comme substances à activités thérapeutiques.

Ainsi ils ont fait l'objet d'une étude quant à leur affinité pour les récepteurs sérotoninergiques du type $5-HT_{1A}$ présents dans l'hippocampe du rat.
Les composés déplacent la liaison d'un ligand spécifique marqué , la ($^3$H]-8-hydroxy-di-n-propylamino-2 tétraline (désignée ci-après par "[$^3$H]-8-OH-DPAT" et décrite par Gozlan et coll., Nature, (1983), 305, 140-142) sur les récepteurs $5-HT_{1A}$.
Les animaux utilisés sont des rats mâles Sprague-Dawley de 160 à 200 g. Après décapitation on en prélève le cerveau et on excise l'hippocampe. On broie le tissu dans un appareil Ultra-Turrax Polytron® pendant 30 s à la moitié de la vitesse maximale dans 10 volumes de tampon Tris 50 mM d'un pH ajusté à 7,4 avec de l'acide chlorhydrique (soit 100 mg de tissu frais par ml). On lave les tissus homogénéisés deux fois à 4°C, en les centrifugeant à chaque fois pendant 10 mn à 48000xg et en remettant le culot en suspension dans du tampon frais refroidi. Finalement on met le dernier culot en suspension dans le tampon pour arriver à une concentration de 50 mg de tissu de départ par ml de tampon à 50 mM. On laisse ensuite incuber à 37°C pendant 10 mn.
On détermine la liaison avec la [$^3$H]-8-OH-DPAT (1 nM) par incubation de 50 µl de suspension de membranes dans un volume final de 250 µl de tampon contenant 10 µM de pargyline et 3 µM de paroxétine. Après une incubation de 15 mn à 37°C on récupère les membranes par filtration sur filtres Whatman GF/B® qu'on lave trois fois avec des quantités aliquotes de 5 ml de tampon glacé. On extrait les filtres dans le liquide de scintillation et on en mesure la radioactivité par scintigraphie liquide. On définit la liaison spécifique de la [$^3$H]-8-OH-DPAT comme la quantité de radioactivité retenue sur les filtres et pouvant être inhibée par co-incubation avec de la 5-hydroxytryptamine à 10 µM. A une concentration de 1 nM de [$^3$H]-8-OH-DPAT la liaison spécifique représente 90% de la radioactivité totale récupérée sur le filtre.
Pour chaque concentration de composé étudié on détermine le pourcentage d'inhibition de la liaison avec la [$^3$H]-8-OH-DPAT, puis la concentration $CI_{50}$, concentration qui inhibe 50% de la liaison.
Pour les composés de l'invention les $CI_{50}$ se situent entre 0,001 et 0,1 µM.

L'activité centrale des composés de l'invention a été évaluée par leurs effets sur les "pointes PGO" (ponto-géniculo-occipitales) induites par la réserpine chez le chat, selon la méthode décrite par H. Depoortere dans Sleep 1976, 3rd Europ. Congr. Sleep Res., Montpellier 1976, 358-361 (Karger, Basel 1977).

On administre des doses cumulatives de composés à étudier (de 0,001 à 3 mg/kg par voie intraveineuse) à des intervalles de temps de 30 mn, 4h après injection intrapéritonéale d'une dose de 0,75 mg/kg de réserpine, à des chats curarisés, sous ventilation artificielle. On recueille les activités électro-encéphalographique et phasique (pointes PGO-R) à l'aide d'électrodes corticales et profondes (genouillé latéral). Pour chaque dose de composé étudié on détermine le pourcentage de diminution du nombre de pointes PGO, puis la $DA_{50}$, dose active qui diminue de 50% ce nombre de pointes.

Pour les composés de l'invention les $DA_{50}$ se situent entre 0,001 et 1 mg/kg par la voie intraveineuse.

Les composés de l'invention ont également fait l'objet d'une étude quant à leur affinité pour les récepteurs dopaminergiques du type $D_2$ présents dans le striatum de rat.

Les composés déplacent la liaison d'un ligand spécifique marqué, la spipérone (désignée ci-après par "$[^3H]$spipérone" et décrite par Briley et Langer., Eur. J. Pharmacol., (1978), 50, 283-284) sur les récepteurs $D_2$.

Les animaux utilisés sont des rats mâles Sprague-Dawley de 150 à 250 g. Après décapitation on en prélève le cerveau et on excise le striatum. On broie le tissu à l'aide d'un broyeur Polytron® dans 50 volumes de tampon Tris-HCl 50 mM contenant du chlorure de sodium (120 mM), du chlorure de potassium (5 mM) et dont le pH est ajusté à 7,4 (soit 100 mg de tissu frais par 5 ml). On lave les tissus homogénéisés deux fois à 4°C, en les centrifugeant à chaque fois pendant 10 mn à 40000xg et en remettant le culot en suspension dans du tampon frais refroidi. Finalement on met le dernier culot en suspension dans le même volume de tampon et on ajoute de l'acide ascorbique (0,1 % en concentration finale) et de la pargyline (10 µM en concentration finale). On laisse ensuite incuber à 37°C pendant 10 mn.

On détermine la liaison de la $[^3H]$spipérone (New England Nuclear activité spécifique 20-40 mCi/mmole) en faisant incuber 100 µl de la suspension membranaire avec le radioligand (0,25 nM) dans un volume final de 1 ml, pendant 20 minutes, à 37°C, en présence ou en l'absence du composé à étudier. On détermine la liaison non spécifique en présence d'halopéridol à la concentration de 10 µM. Après incubation, on récupère les membranes par filtration sur filtres Whatman GF/B® qu'on lave avec deux volumes de 5 ml de tampon glacé. On extrait les filtres dans le liquide de scintillation et on en mesure la radioactivité par scintigraphie liquide avec une efficacité de 50 à 60 %. Pour chaque composé testé, on exprime les résultats par la $CI_{50}$, c'est à dire par la concentration qui inhibe 50 % de la liaison de la $[^3H]$spipérone, calculée par une méthode graphique ou mathématique.

Pour les composés de l'invention les $CI_{50}$ se situent entre 0,020 et 0,2 µM.

Les résultats des essais montrent que les composés de formule (I) possèdent, *in vitro*, une grande affinité pour les récepteurs sérotoninergiques de type $5\text{-}HT_{1A}$ et les récepteurs dopaminergiques de type $D_2$.

*In vivo* ils montrent une activité agoniste, agoniste partielle, ou antagoniste, au niveau des récepteurs sérotoninergiques de type $5\text{-}HT_{1A}$ .

Les composés de l'invention peuvent donc être utilisés notamment pour le traitement des psychoses, des états d'anxiété, des troubles du sommeil, pour la régulation de la prise de nourriture, pour le traitement ou la prévention des vomissements et du mal des transports, pour le traitement de désordres vasculaires ou cardiovasculaires tels que la migraine et l'hypertension.

A cet effet ils peuvent être présentés sous toutes formes appropriées à leur administration par voie orale ou parentérale, associés à tous excipients convenables, et dosés pour permettre une posologie journalière de 1 à 1000 mg.

## Revendications

**1.** Composés de formule générale (I)

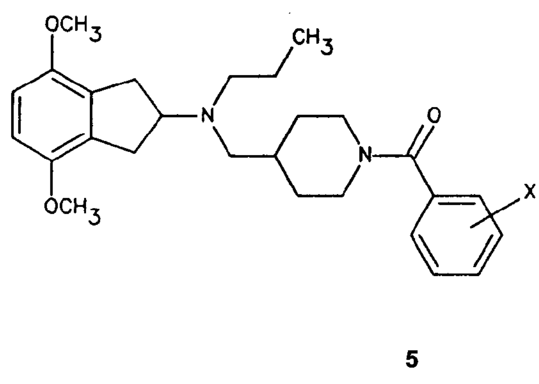

(I)

dans laquelle X représente un atome de chlore ou un groupe méthyle, méthoxy ou éthoxy, à l'état de bases libres ou de sels d'addition à des acides.

2. Procédé de préparation des composés selon la revendication 1, caractérisé en ce qu'on fait réagir d'abord la 4,7-diméthoxyindan-2-amine, éventuellement sous forme de sel d'addition avec un acide, avec le chlorure de propionyle, dans un solvant aprotique inerte ou dans un solvant halogéné, et en présence d'une base organique, puis on réduit le *N*-(4,7-diméthoxyindan-2-yl)propanamide intermédiaire, ainsi obtenu, pour obtenir la 4,7-diméthoxy-*N*-propyl-indan-2-amine, et on fait ensuite réagir celle-ci avec un tosylate de formule générale (IV)

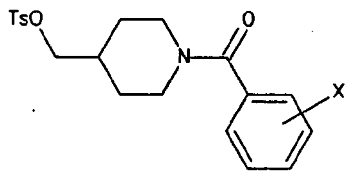

(IV)

(dans laquelle Ts représente un groupe (4-méthylphényl)sulfonyle et X est tel que défini dans la revendication 1), soit en l'absence, soit en présence d'un solvant inerte, à une température de 20 à 150°C, et éventuellement en présence d'une base organique ou minérale.

3. Médicament caractérisé en ce qu'il consiste en un composé selon la revendication 1.

4. Composition pharmaceutique caractérisée en ce qu'elle contient un composé selon la revendication 1 associé à un excipient pharmaceutique.

**Revendications pour les Etats contractants suivants : ES, GR**

1. Procédé de préparation des composés de formule générale (I)

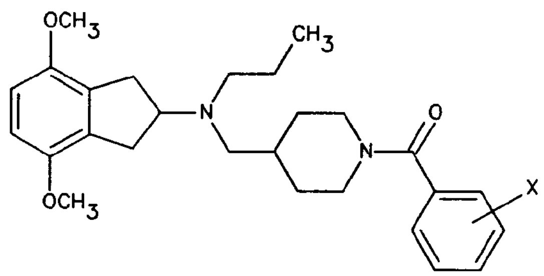

(I)

dans laquelle X représente un atome de chlore ou un groupe méthyle, méthoxy ou éthoxy,
procédé caractérisé en ce qu'on fait réagir d'abord la 4,7-diméthoxyindan-2-amine, éventuellement sous forme de sel d'addition avec un acide, avec le chlorure de propionyle, dans un solvant aprotique inerte ou dans un solvant halogéné, et en présence d'une base organique, puis on réduit le *N*-(4,7-diméthoxyindan-2-yl)propanamide intermédiaire, ainsi obtenu, pour obtenir la 4,7-diméthoxy-*N*-propyl-indan-2-amine, et on fait ensuite réagir celle-ci avec un tosylate de formule générale (IV)

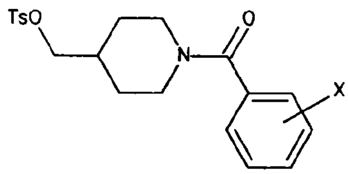

(IV)

(dans laquelle Ts représente un groupe (4-méthylphényl)sulfonyle et X est tel que défini dans la revendication 1), soit en l'absence, soit en présence d'un solvant inerte, à une température de 20 à 150°C, et éventuellement en présence d'une base organique ou minérale.

Office européen des brevets

# RAPPORT DE RECHERCHE EUROPEENNE

Numero de la demande

EP 92 40 2440
Page 1

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| A,D | NATURE<br>vol. 305, 8 Septembre 1983,<br>pages 140 - 142<br>H. GOZLAN ET. AL. 'IDENTIFICATION OF PRESYNAPTIC SEROTONIN AUTORECEPTORS USING A NEW LIGAND: 3H-PAT'<br>* le document en entier *<br>--- | 1-4 | C07D211/26<br>A61K31/445 |
| A | EP-A-0 343 830 (ELI LILLY AND COMPANY)<br>29 Novembre 1989<br>* exemple 10 *<br>* revendication 1 *<br>--- | 1-4 | |
| A | JOURNAL OF MEDICINAL CHEMISTRY.<br>vol. 25, no. 2, 1982, WASHINGTON US<br>pages 136 - 141<br>DEMARINIS ET. AL. 'ALPHA-ADRENERGIC AGENTS'<br>* le document en entier *<br>--- | 1-4 | |
| A | JOURNAL OF MEDICINAL CHEMISTRY.<br>vol. 32, no. 8, 1989, WASHINGTON US<br>pages 1959 - 1962<br>CANNON ET. AL. '5-HT1A-RECEPTOR ANTAGONISM'<br>* le document en entier *<br>--- | 1-4 | **DOMAINES TECHNIQUES RECHERCHES (Int. Cl.5 )**<br>C07D<br>A61K |
| A | MOLECULAR PHARMACOLOGY<br>vol. 16, no. 3, 3 Novembre 1979,<br>pages 687 - 699<br>S. J. PEROUTKA, S. H. SNYDER 'MULTIPLE SEROTONIN RECEPTORS'<br>* le document en entier *<br>--- | 1-4 | |
| P,A | EP-A-0 447 292 (SYNTHELABO)<br>18 Septembre 1991<br>* le document en entier *<br>---<br>-/-- | 1-4 | |

Le présent rapport a été établi pour toutes les revendications

| Lieu de la recherche | Date d'achèvement de la recherche | Examinateur |
|---|---|---|
| LA HAYE | 13 OCTOBRE 1992 | Bernd Kissler |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)

Office européen des brevets

# RAPPORT DE RECHERCHE EUROPEENNE

Numero de la demande

EP 92 40 2440
Page 2

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| A | JOURNAL OF MEDICINAL CHEMISTRY.<br>vol. 30, no. 1, 1987, WASHINGTON US<br>pages 1 - 12<br>R. A. GLENNON 'Central Serotonin Receptors as Targets for Drug Research'<br>* le document en entier *<br>--- | 1-4 | |
| A | JOURNAL OF MEDICINAL CHEMISTRY.<br>vol. 25, no. 7, 1982, WASHINGTON US<br>pages 858 - 864<br>R. D. SINDELAR ET. AL.<br>'2-Amino-4,7-dimethoxyindan derivatives.'<br>----- | 1-4 | |

DOMAINES TECHNIQUES RECHERCHES (Int. Cl.5 )

Le présent rapport a été établi pour toutes les revendications

| Lieu de la recherche | Date d'achèvement de la recherche | Examinateur |
|---|---|---|
| LA HAYE | 13 OCTOBRE 1992 | Bernd Kissler |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)